# EUROPEAN PATENT APPLICATION

(11) **EP 2 609 921 A1**
(43) Date of publication of application: **03.07.2013**
(21) Application number: 13161401.8
(22) Date of filing: 25.02.2010
(51) Int. Cl.: A61K 31/58, A61P 7/10

(54) **Treatment of pervasive developmental disorders with tocotrienols or tocotrienol enriched extracts**

(30) Priority: 25.06.2009 US 269627 P
(62) Divisional of application: 10792467.2
(71) Applicant: Ampere Life Sciences, Inc., Mountain View, CA 94043 (US)
(72) Inventor: Miller, Guy M., Mountain View, CA California 94043 (US)
(74) Representative: Sexton, Jane Helen

(57) **Abstract**

Methods of treating or suppressing Pervasive Developmental Disorders (PDDs) including; Autistic Disorder, Asperger's Disorder, Childhood Disintegrative Disorder (CDD), Rett's Disorder, and PDD-Not Otherwise Specified (PDD-NOS) comprising administering to a subject in need thereof a therapeutically effective amount of a tocotrienol, a tocotrienol ester, a tocotrienol ether, a tocotrienol enriched extract or mixtures thereof.

## Description

### CROSS-REFERENCE TO RELATED APPLICATIONS

This application claims priority benefit of United States Provisional Patent Application No. 61/269,627 filed June 25, 2009. The content of that application is hereby incorporated by reference herein in its entirety.

### FIELD OF THE INVENTION

The application discloses methods useful for treatment, prevention, or suppression of diseases, developmental delays, and symptoms of Pervasive Developmental Disorders including Autistic Spectrum Disorders, with tocotrienols, tocotrienol esters, tocotrienol ethers, tocotrienol enriched extracts, or mixtures thereof.

### BACKGROUND OF THE INVENTION

Pervasive Developmental Disorder (PDD) is a category of neurological disorders characterized by severe and pervasive impairment in several areas of development, including social interaction and communications skills. The five disorders under PDD are Autistic Disorder, Asperger's Disorder, Childhood Disintegrative Disorder (CDD), Rett's Disorder, and PDD-Not Otherwise Specified (PDD-NOS). Specific diagnostic criteria for each of these disorders can be found in the Diagnostic & Statistical Manual of Mental Disorders (DSM-IV-TR) as distributed by the American Psychiatric Association (APA). Autistic Spectrum Disorder (ASD) is an umbrella term that is used to represent a broad heterogeneous disorder by collectively grouping autistic disorder, Asperger's Disorder and PDD-NOS.

Autism, the most common of the Pervasive Developmental Disorders, affects an estimated 1 in approximately 150 births. Estimates of the prevalence of ASD are in the range of 6.5 to 6.6 per 1000 based on Autism and Developmental Disabilities Monitoring Network Surveillance (Year 2002). Indeed, as of 2003-2004, as many as 1.5 million Americans are believed to have some form of autism. Autism is a childhood encephalopathy characterized by deficiencies in social interaction and communication and by repetitive and stereotyped behaviors. Based on statistics from the U.S. Department of Education and other governmental agencies; autism is growing at a rate of 10-17 percent per year. At these rates, the Autism Society of America (ASA) estimates that the prevalence of autism could easily reach 4 million Americans in the next decade.

Of the other four PDD forms, Asperger's syndrome is closest to autism in signs and likely causes; Rett's disorder and childhood disintegrative disorder share several signs with autism, but may have unrelated causes; PDD not otherwise specified (PDD-NOS) is diagnosed when the criteria are not met for a more specific disorder (Lord C, et al. "Autism spectrum disorders" Neuron (2000) 28 (2): 355-63).

Autism is a complex serious developmental disability that interferes with, among other things, the normal development of the brain in the areas of social interaction and communication skills and which causes severely restricted interests and repetitive behavior. Typically, autistic children and adults have difficulties in verbal and non-verbal communication, social interactions, and leisure or play activities. Autism can include language disorders with impaired understanding, echolalia, pronominal reversal (such as using "you" instead of "I" or "me" when referring to one's self), rituals and compulsive phenomena, and uneven intellectual development with mental retardation. Autistic children are also at increased risk of developing seizure disorders, especially during their teen years. Autism typically appears during the first three years of life and is the result of a neurological disorder that affects the functioning of the brain.

The overall incidence of autism is, for the most part, globally consistent. Indeed, autism knows no racial, ethnic, or social boundaries, and family income, lifestyle, and educational levels do not affect the chance of autism's occurrence. However, it has been found to be four times more prevalent in boys than girls. On the other hand, Rett's Disorder is more prevalent in girls than boys.

Since being first described by Dr. Leo Kanner in 1943, the understanding of autism has grown tremendously. Although autism is defined by a certain set of behaviors, it is a spectrum disorder in that its symptoms and characteristics can be present in a wide variety of combinations, from mild to severe. Therefore, autistic children and adults can exhibit any combination of the behaviors in any degree of severity. Two individuals, both with the same diagnosis, may have varying skills and display very different actions. Those only mildly affected may exhibit slight delays in language or communication and may face greater challenges in social interactions. For example, one may have difficulty initiating and/or maintaining a conversation. Communication by autistic children or adults is often displayed as talking at others (for example, a monologue on a favorite subject that continues despite attempts by others to interject comments).

Autism seems to cause those affected by it to process and respond to information in unique ways. In some individuals with PDD including Autism, aggressive and/or self-injurious behavior may exist. The following traits, as identified by the ASA, may also be present in persons with autism: insistence on sameness or resistance to change; difficulty in expressing needs; (i.e. uses gestures or pointing instead of words); repeating words or phrases in place of normal, responsive language; laughing, crying, or showing distress for reasons not apparent to others; preferring to be alone or an aloof manner; tantrums; difficulty in mixing with others; may not wanting to cuddle or be cuddled; little or no eye contact; unresponsive to normal teaching methods; sustained odd play; spinning objects; inappropriate attachments to objects; apparent over-sensitivity or under-sensitivity to pain; no real fears of danger; marked physical over-activity or extreme under-activity; uneven gross/fine motor skills; and/or non-responsiveness to verbal cues (i.e. acts as if deaf although hearing tests in normal range).

People with autism have social impairments that appear early in childhood and continue through adulthood. Autistic infants show less attention to social stimuli, smile and look at others less often, and respond less to their own name. Autistic toddlers have more striking social deviance; for example, they have less eye contact and anticipatory postures and are more likely to communicate by manipulating another person's hand (Volkmar F, et al "Autism in infancy and early childhood" Annu Rev Psychol (2005) 56: 315-36.) Three- to five-year-old autistic children are less likely to exhibit social understanding, approach others spontaneously, imitate and respond to emotions, communicate nonverbally, and take turns with others. However, they do form attachments to their primary caregivers. (Sigman M, et al. "Early detection of core deficits in autism" Ment Retard Dev Disabil Res Rev. (2004) 10 (4): 221-33). They display moderately less attachment security than usual, although this feature disappears in children with higher mental development or less severe autism spectrum disorders. Older children and adults with ASD perform worse on tests of face and emotion recognition (Sigman M. *et al,* see supra). Contrary to common belief, autistic children do not prefer to be alone. Making and maintaining friendships often proves to be difficult for those with autism. For them, the quality of friendships, not the number of friends, predicts how lonely they are.

Unlike those with autism, people with Asperger's syndrome are not usually withdrawn around others; they approach others, even if awkwardly, for example by engaging in a one-sided, long-winded speech about a favorite topic while being oblivious to the listener's feelings or reactions, such as signs of boredom or haste to leave.

About a third to a half of individuals with autism does not develop enough natural speech to meet their daily communication needs; (Noens I, et al, "The ComFor: an instrument for the indication of augmentative communication in people with autism and intellectual disability". J Intellect Disabil Res (2006) 50 (9): 621-32.) Differences in communication may be present from the first year of life, and may include delayed onset of babbling, unusual gestures, diminished responsiveness, and the desynchronization of vocal patterns with the caregiver. In the second and third years, autistic children have less frequent and less diverse babbling, consonants, words, and word combinations; their gestures are less often integrated with words. Autistic children are less likely to make requests or share experiences, and are more likely to simply repeat others' words or reverse pronouns.

For individuals with autism, sensory integration problems are common. In particular, their senses may be either over- or under-active. The fuzz of a kiwi may actually be experienced as painful; a sweet, fruity smell may cause a gagging reflex. Some children or adults with autism are particularly sensitive to sound, so that even the most ordinary daily noises are painful.

Although there is no single known cause for autism, it is generally accepted that it is caused by abnormalities in brain structure or function. The shape and structure of the brain in autistic versus non-autistic children show differences when brain scans are viewed. Currently the links between heredity, genetics and medical problems are being investigated by researchers, as well as a number of other theories. The theory of a genetic basis of the disorder is supported by the fact that, in many families, there appears to be a pattern of autism or related disabilities. While no one gene has been identified as causing autism, researchers are searching for irregular segments of genetic code that autistic children may have inherited. While researchers have not yet identified a single trigger that causes autism to develop, it also appears that some children are born with a susceptibility to autism.

Other researchers are investigating the possibility that under certain conditions, a cluster of unstable genes may interfere with brain development resulting in autism. Still other researchers are investigating problems during pregnancy or delivery as well as environmental factors such as viral infections, metabolic imbalances, and exposure to environmental chemicals. Yet other researchers are investigating the link between autism and chemical toxicity, in particular with the mercury-containing vaccine preservative thimerosal.

Some cases of autism have been associated with several different organic conditions, including bioenergetic metabolism deficiency suggested by the detection of high lactate levels in some patients (Coleman M. et al, Autism and Lactic Acidosis, J. Autism Dev Disord., (1985) 15: 1-8; Laszlo et al Serum serotonin, lactate and pyruvate levels in infantile autistic children, Clin. Chim. Acta (1994) 229:205-207; and Chugani et al., Evidence of altered energy metabolism in autistic children, Progr. Neuropsychopharmacol Biol Psychiat., (1999) 23:635-641) and by nuclear magnetic resonance imagining as well as positron emission tomography scanning which documented abnormalities in brain metabolism. Although the mechanism of hyperlactacidemia remains unknown, a likely possibility involves mitochondrial oxidative phosphorylation dysfunction in neuronal cells. A small subset of autistic patients diagnosed with deficiencies in complex I or III of the respiratory chain have been reported in the literature (see Oliveira, G., Developmental Medicine & Child Neurology (2005) 47 185-189; and Filipek, PA et al., Journal of Autism and Developmental Disorders (2004) 34:615-623.) However, in many of the cases of autism where there is some evidence of mitochondrial dysfunction, there is an absence of the classic features associated with mitochondrial disease, such as mitochondrial pathology in muscle biopsy (see Rossignol, D.A. et al., Am J. Biochem. & Biotech, 4 (2) 208-217).

The main goals of treatment are to lessen associated deficits and family distress, and to increase quality of life and functional independence. No single treatment is best and treatment is typically tailored to the child's needs. Intensive, sustained special education programs and behavior therapy early in life can help children acquire self-care, social, and job skills, (Myers SM, et al. "Management of children with autism spectrum disorders" Pediatrics (2007) 120 (5): 1162-82) and Angley M, et al. "Children and autism-part 1-recognition and pharmacological management" Aust Fam. Physician (2007) 36 (9): 741-4) and often improve functioning and decrease symptom severity and maladaptive behaviors ; (Rogers SJ, et al., . "Evidence-based comprehensive treatments for early autism" J Clin. Child Adolesc. Psychol. (2008) 37 (1): 8-38).

Medications have not been proven to correct deficits of ASDs and are not the primary treatment. They are used to treat problems associated with autism disorders, such as associated maladaptive behaviors or psychiatric comorbidities that may interfere with educational progress, socialization, health or safety and quality of life. More than half of U.S. children diagnosed with autistic disorders are prescribed psychoactive drugs or anticonvulsants, with the most common drug classes being antidepressants, stimulants, and antipsychotics.(Oswald DP, et al. "Medication use among children with autism spectrum disorders". J Child Adolesc Psychopharmacol (2007) 17 (3): 348-55.) Aside from antipsychotics, there is scant reliable research about the effectiveness or safety of drug treatments for children, adolescents or adults with ASD. A person with ASD may respond atypically to medications, the medications can have adverse effects, and no known medication relieves autism's core symptoms of social and communication impairments. Alternative nutritional therapies for autistic children may include Idebenone and CoQ10, because of their superior antioxidant properties, but no studies have been performed to prove their efficacy.

US Patent Publication 2005/0203066 discloses compounds, compositions and methods for treatment of developmental delay in cognitive, motor, language, executive function or social skills with a pyrimidine nucleotide precursor, but it does not disclose any compounds, compositions or methods of treatment with compounds of the present invention.

Krajcovicova-Kudlackova et al, Bratisl Lek Listy, (2009), 110(4): 247-250 have shown that plasma concentrations of exogenous antioxidants such as Vitamin E and A and lycopene in autistic subjects are insufficient.

Co-owned US provisional patent application USAN 61/191,696 titled "Treatment of Pervasive Development Disorders with Redox-active Therapeutics," Patent Cooperation Treaty application PCT/US2009/056254, and U.S. patent application USAN 12/555,700 disclose the use of certain redox-active compounds for the treatment or suppression of pervasive development disorders.

Thus, there is an unmet need for improved methods of treating patients exhibiting symptoms of Pervasive Developmental Disorders, particularly symptoms of autism with therapies that are safe and can be easily administered to children.

### SUMMARY OF THE INVENTION

The present invention provides methods that can reduce, ameliorate or treat the symptoms of Pervasive Developmental Disorders, particularly symptoms of autism in a human patient. Briefly, the methods and compositions comprise administering a physiologically or therapeutically effective amount of a tocotrienol, a tocotrienol ester, a tocotrienol ether, a tocotrienol enriched extract or composition, or mixtures thereof, in sufficient quantities to reduce, ameliorate or treat at least one of the symptoms of Pervasive Developmental Disorders, particularly symptoms of autism, preferably all of the symptoms of Pervasive Developmental Disorders, particularly of autism. When administered to human patients suffering from Pervasive Developmental Disorders, particularly from autism, without restriction on the normal diet of the patients, the compositions and methods may reduce or improve one or more symptoms of Pervasive Developmental Disorders, particularly symptoms of autism, such as increased eye contact, better enunciation and use of pronouns, less fatigue, singing a song with the melody and words together and the entire song understandable, playing with age appropriate friends, fewer tantrums, better sleep patterns, improved politeness and coordination, being more loving, acknowledging another individual's emotion, and increased voice and word association. In some embodiments the composition comprises alpha-tocotrienol or esters or ethers or mixtures thereof, particularly the formulation comprises alpha-tocotrienol. In other embodiments, the composition comprises a tocotrienol enriched extract.

In another aspect, the invention embraces a method of reducing, ameliorating or treating the symptoms associated with, or of treating or suppressing Pervasive Developmental Disorder (PDD), including of Autistic Disorder, Asperger's Disorder, Childhood Disintegrative Disorder (CDD), Rett's Disorder, and PDD-Not Otherwise Specified (PDD-NOS) in a patient in need of such treatment by administering a therapeutically or physiologically effective amount of formulation comprises a therapeutically effective amount of one or more agents selected from the group consisting of alpha-tocotrienol, beta-tocotrienol, gamma-tocotrienol, delta-tocotrienol, or mixtures thereof.

In some embodiments, the tocotrienol is essentially pure alpha-tocotrienol. In other embodiments the tocotrienol is an alpha-tocotrienol enriched composition of tocotrienols also optionally including beta-tocotrienol, delta-tocotrienol and gamma-tocotrienol, but with less than 40%, less than 30%, less than 25%, less than 20%, less than 15%, less than 10%, less than 5% or less than 2% tocopherol or no detectable tocopherol. In some embodiments, the tocotrienol is alpha-tocotrienol essentially free of tocopherol. In the spirit of the invention, "essentially free of tocopherol" means less than 20%, less than 15%, less than 10%, less than 5% or less than 2% of tocopherol of the total tocotrienol/tocopherol weight. In other embodiments the tocotrienol is an alpha-tocotrienol enriched composition of tocotrienols also optionally including beta-tocotrienol, delta-tocotrienol and gamma-tocotrienol, but with less than 40%, less than 30%, less than 25%, less than 20%, less than 15%, less than 10%, less than 5% or less than 2% alpha-tocopherol or no detectable alpha-tocopherol. In some embodiments, the tocotrienol is alpha-tocotrienol essentially free of alpha-tocopherol. In the spirit of the invention, "essentially free of alpha-tocopherol" means less than 20%, less than 15%, less than 10%, less than 5% or less than 2% of alpha-tocopherol of the total tocotrienol/tocopherol weight.

In another aspect, the invention embraces a method of reducing, ameliorating or treating the symptoms associated with, or of treating or suppressing Pervasive Developmental Disorder (PDD), including of Autistic Disorder, Asperger's Disorder, Childhood Disintegrative Disorder (CDD), Rett's Disorder, and PDD-Not Otherwise Specified (PDD-NOS) in a patient in need of such treatment by administering a therapeutically or physiologically effective amount of one or more agents selected from the group consisting of alpha-tocotrienol esters, beta-tocotrienol esters, gamma-tocotrienol esters, delta-tocotrienol esters, and mixtures thereof.

In another aspect, the invention embraces a method of reducing, ameliorating or treating the symptoms associated with, or of treating or suppressing Pervasive Developmental Disorder (PDD), including of Autistic Disorder, Asperger's Disorder, Childhood Disintegrative Disorder (CDD), Rett's Disorder, and PDD-Not Otherwise Specified (PDD-NOS) in a patient in need of such treatment by administering a therapeutically or physiologically effective amount of one or more agents selected from the group consisting of alpha-tocotrienol ethers, beta-tocotrienol ethers, gamma-tocotrienol ethers, delta-tocotrienol ethers, and mixtures thereof. In some embodiments, the tocotrienol ether is a polyethoxylated tocotrienol. In some embodiments, the tocotrienol ether is a polyethoxylated alpha-tocotrienol ether, a polyethoxylated beta-tocotrienol ether, a polyethoxylated gamma-tocotrienol ether, a polyethoxylated delta-tocotrienol ether, and mixtures thereof.

In some embodiments, the tocotrienol ester is selected from the group consisting of an acetic acid ester, a nicotinic acid ester, a phosphate ester, a glutamic acid ester, a linoleic acid ester, a palmitic acid ester, an aspartic ester, and a succinic acid ester. In other embodiments, the tocotrienol is a polyethoxylated tocotrienol. In other embodiments the tocotrienol is a tocotrienol polyethylene glycol succinate derivative. In other embodiments, including any of the foregoing embodiments, the tocotrienol ester is alpha-tocotrienol ester.

In other embodiments, the tocotrienol ester is selected from alpha-tocotrienol succinate, beta-tocotrienol succinate, gamma-tocotrienol succinate, delta-tocotrienol succinate; alpha-tocotrienol acetate, beta-tocotrienol acetate, gamma-tocotrienol acetate, delta-tocotrienol acetate; alpha-tocotrienol nicotinate, beta-tocotrienol nicotinate, gamma-tocotrienol nicotinate, delta-tocotrienol nicotinate; alpha-tocotrienol phosphate, beta-tocotrienol phosphate, gamma-tocotrienol phosphate, delta-tocotrienol phosphate, alpha-tocotrienol glutamate, beta-tocotrienol glutamate, gamma-tocotrienol glutamate, delta-tocotrienol glutamate, alpha-tocotrienol linoleate, beta-tocotrienol linoleate, gamma-tocotrienol linoleate, delta-tocotrienol linoleate, alpha-tocotrienol palmitate, beta-tocotrienol palmitate, gamma-tocotrienol palmitate, delta-tocotrienol palmitate, alpha-tocotrienol aspartate, beta-tocotrienol aspartate, gamma-tocotrienol aspartate, and delta-tocotrienol aspartate.

In particular embodiments, the tocotrienol ester is selected from alpha-tocotrienol succinate, alpha-tocotrienol acetate, alpha-tocotrienol nicotinate, alpha-tocotrienol phosphate, alpha-tocotrienol glutamate, alpha-tocotrienol linoleate, alpha-tocotrienol palmitate, and alpha-tocotrienol aspartate. In another embodiment, the tocotrienol ester is alpha-tocotrienol acetate. In another embodiment, the tocotrienol ester is alpha-tocotrienol succinate. In another embodiment, the tocotrienol ester is alpha-tocotrienol nicotinate. In another embodiment, the tocotrienol ester is alpha-tocotrienol glutamate. In another embodiment, the tocotrienol ester is alpha-tocotrienol phosphate. In another embodiment, the tocotrienol ester is alpha-tocotrienol palmitate. In another embodiment, the tocotrienol ester is alpha-tocotrienol linoleate. In another embodiment, the tocotrienol ester is alpha-tocotrienol aspartate.

In other embodiments, the tocotrienol ester is a polyethoxylated alpha-tocotrienol, polyethoxylated beta-tocotrienol, polyethoxylated gamma-tocotrienol, or polyethoxylated delta-tocotrienol. In some embodiments, the tocotrienol ester is polyethoxylated alpha-tocotrienol. In other embodiments, the tocotrienol ester is alpha-tocotrienol polyethylene glycol succinate.

In another aspect, the invention embraces a method of reducing, ameliorating or treating the symptoms associated with, or of treating or suppressing Pervasive Developmental Disorder (PDD), including of Autistic Disorder, Asperger's Disorder, Childhood Disintegrative Disorder (CDD), Rett's Disorder, and PDD-Not Otherwise Specified (PDD-NOS) in a patient in need of such treatment by administering a therapeutically or physiologically effective amount of formulation comprising a therapeutically effective amount of a an enriched tocotrienol extract of palm oil, rice bran oil, barley or annatto. In another embodiment the formulation of the present invention comprises an enriched tocotrienol extract from palm oil, as sold by Carotech, Golden Hope Bioorganic, Carotech, Davos Life Science, Beijing Gingko Group, Eisai, Eastman Corporation or Palm Nutraceuticals. In some embodiments, and any of the foregoing embodiments the tocotrienol extract is Tocomin-50^{®}. In another embodiment, the formulation of the present invention comprises an enriched tocotrienol extract from annatto.

In some of the foregoing embodiments, the symptoms treated by the compounds of the present invention are selected from the group of symptoms consisting of eye contact avoidance, failure to socialize, attention deficit, poor mood, hyperactivity, anxiety, stimming, poor comprehension, inappropriate speech, abnormal sound sensitivity, poor digestion, disrupted sleep, and perseveration, and where the decreased incidence is measured relative to the incidence in the untreated individual.

In one embodiment, the present invention provides pharmaceutical or nutraceutical compositions able to reduce the symptoms of autism in a patient, comprising a physiologically effective amount of a tocotrienol, a tocotrienol ester, a tocotrienol ether, a tocotrienol enriched extract or composition, or mixtures thereof and a physiologically acceptable carrier, adjuvant, excipient, buffer and diluent.

In still a further aspect, the present invention provides foods, medical foods, dietary foods, functional foods, food supplements, or dietary supplements comprising compositions of a tocotrienol, a tocotrienol ester, a tocotrienol ether, a tocotrienol enriched extract or composition, or mixtures thereof, and at least one of the group consisting of a physiologically or nutritionally acceptable carrier, adjuvant, excipient, buffer and diluent.

In yet another aspect, the invention also provides articles of manufacture and kits containing materials useful for treating or suppressing autistic spectrum disorder. The invention also provides kits comprising a tocotrienol, a tocotrienol ester, a tocotrienol ether, a tocotrienol enriched extract or composition, or mixtures thereof. In some embodiments, the kit of the invention comprises a container suitable for storing a tocotrienol, a tocotrienol ester, a tocotrienol ether, a tocotrienol enriched extract or composition, or mixtures thereof. In some embodiments, the kit of the invention comprises a container suitable for storing a tocotrienol, a tocotrienol ester, a tocotrienol ether, a tocotrienol enriched extract or composition, or mixtures thereof, and at least one of the group consisting of a physiologically or nutritionally acceptable carrier, adjuvant, excipient, buffer and diluent.

In other aspects, the kits may be used for any of the methods described herein, including, for example, to treat an individual with ASD disorder, or to suppress an ASD disorder in an individual.

The present invention comprises multiple aspects, features and embodiments; where such multiple aspects, features and embodiments can be combined and permuted in any desired manner.

These and other aspects, features and embodiments of the present invention will become evident upon reference to the remainder of this application, including the following detailed description. In addition, various references are set forth herein that describe in more detail certain compositions, and/or methods; all such references are incorporated herein by reference in their entirety.

### DETAILED DESCRIPTION OF THE INVENTION

The present invention provides methods and compositions able to reduce the symptoms of Pervasive Developmental Disorder including autistic spectrum disorder in a patient. Briefly, the compositions and methods comprise administering a tocotrienol, a tocotrienol ester, a tocotrienol ether, a tocotrienol enriched extract or composition, or mixtures thereof to a human patient in sufficient quantities to reduce the effects of the autistic disease. An initial trial wherein a tocotrienol, a tocotrienol ester, a tocotrienol ether, a tocotrienol enriched extract or composition, or mixtures thereof are administered to human patients, without restriction on the normal diet of the patients, would provide a significant number of the patients with a significant reduction of one or more symptoms, such as increased eye contact, better enunciation and use of pronouns, less fatigue, singing a song with the melody and words together and the entire song understandable, playing with age appropriate friends, fewer tantrums, better sleep patterns, improved politeness and coordination, being more loving, acknowledging another individual's emotion, increased voice and word association.

"Subject," "individual," or "patient" are used interchangeably, and refer to a mammal, preferably a human.

"Treating" a disease with the compounds and methods discussed herein is defined as administering one or more of the compounds discussed herein, with or without additional therapeutic agents, in order to reduce or eliminate either the disease or one or more symptoms of the disease, or to retard the progression of the disease or of one or more symptoms of the disease, or to reduce the severity of the disease or of one or more symptoms of the disease. "Suppression" of a disease with the compounds and methods discussed herein is defined as administering one or more of the compounds discussed herein, with or without additional therapeutic agents, in order to suppress the clinical manifestation of the disease, or to suppress the manifestation of adverse symptoms of the disease. The distinction between treatment and suppression is that treatment occurs after adverse symptoms of the disease are manifest in a subject, while suppression occurs before adverse symptoms of the disease are manifest in a subject. Suppression may be partial, substantially total, or total. Because the autism disorders are inherited, genetic screening can be used to identify patients at risk of the disease. The compounds and methods of the invention can then be administered to asymptomatic patients at risk of developing the clinical symptoms of the disease, in order to suppress the appearance of any adverse symptoms.

"Therapeutic use" of the compounds discussed herein is defined as using one or more of the compounds discussed herein to treat or suppress a disease, as defined above. A "therapeutically effective amount" of a compound is an amount of the compound, which, when administered to a subject, is sufficient to reduce or eliminate either a disease or one or more symptoms of a disease, or to retard the progression of a disease or of one or more symptoms of a disease, or to reduce the severity of a disease or of one or more symptoms of a disease, or to suppress the clinical manifestation of a disease, or to suppress the manifestation of adverse symptoms of a disease. A therapeutically effective amount can be given in one or more administrations.

A "physiologically effective amount" of an active substance indicates an adequate amount of the active substances to have a significant, externally observable effect on the patient. Thus, such a physiologically effective amount affects one or more of the characteristics in the patient without the need for special equipment to determine the effect. For example, a physiologically effective amount of a compound of the present invention has a significant, externally observable effect on the behavior of the patient by reducing one or more of the symptoms of autism or other pervasive developmental disorder. Accordingly, one can determine whether an adequate amount of the active substance has been administered by watching the patient and observing whether changes have occurred in the patient due to the active substance.

Esters of tocotrienols with aspartate can be esterified via the alpha-carboxy group of aspartate, the side-chain (gamma-carboxy) group of aspartate, or tocotrienols esterified at the alpha-carboxy group of aspartate can be mixed with tocotrienols esterified at the side-chain carboxy group of aspartate in any ratio. Esters of tocotrienols with glutamate can be esterified via the alpha-carboxy group of glutamate, the side-chain (delta-carboxy) group of glutamate, or tocotrienols esterified at the alpha-carboxy group of glutamate can be mixed with tocotrienols esterified at the side-chain carboxy group of glutamate in any ratio.

The invention also includes all stereoisomers of the compounds and their use in the methods, including diastereomers and enantiomers. The invention also includes mixtures of stereoisomers in any ratio, including, but not limited to, racemic mixtures, and their use in the methods.

In a first aspect, the present invention provides compositions comprising a tocotrienol, a tocotrienol ester, a tocotrienol ether, a tocotrienol enriched extract or composition, or mixtures thereof that are able to reduce the symptoms of Pervasive Developmental Disorder (PDD), including of Autistic Disorder, Asperger's Disorder, Childhood Disintegrative Disorder (CDD), Rett's Disorder, and PDD-Not Otherwise Specified (PDD-NOS) in a human patient. For example, the compositions are able to reduce one or more symptoms, such as increased eye contact, better enunciation and use of pronouns, less fatigue, fewer tantrums, better sleep patterns, improved politeness and coordination, and increased voice and word association. In other words, the compositions are able to effect an adequate reduction of one or more of the observable characteristics of autism by an amount that is observable to a human observer, such as a parent, physician or caretaker, without the use of special devices such as microscopes or chemical analytical devices. The compositions reduce such symptoms by providing a physiologically effective amount of a tocotrienol, a tocotrienol ester, a tocotrienol ether, a tocotrienol enriched extract or composition, or mixtures thereof and at least one of the group consisting of a physiologically acceptable carrier, adjuvant, excipient, buffer and diluent, which terms are used in their ordinary sense to indicate substances that assist in the packaging, delivery, absorption, or the physiological effect of the compounds. The physiologically acceptable carriers, adjuvants, excipients, buffers and diluents are preferably nontoxic to recipients at the dosages and concentrations employed. Representative samples include oil, water, isotonic saline solutions that are preferably buffered at physiological pH (such as phosphate-buffered saline or Tris-buffered saline), mannitol, dextrose, glycerol, and ethanol, as well as selected polypeptides or proteins such as human serum albumin, maltodextrin, L-lysine, lactase and other carbohydratases, lipase and non-specific proteases such as papain. The carrier, adjuvant, excipient, buffer, or diluent may be combined with the compositions disclosed herein to provide compositions either as liquid solutions or, preferably, in solid form. For example, when the compositions are to be administered orally, the compositions may be produced in any of powder, tablet or capsule form. The compositions can also be administered as elixirs, liquids, solutions, suspension, emulsions, soft gelatin capsules, and hard gelatin capsules. The preparation of the above dosage forms are well known in the art.

The compositions of the present invention are preferably administered orally, but may also be administered via other direct routes, such as rectal or, in the case of pharmaceutically designed compositions, via transcutaneous methods such as intraarterial, intramuscular, intraperitoneal, subcutaneous, intraocular, and intravenous. Other routes such as buccal/sublingual, nasal, topical (such as transdermal), pulmonary and central nervous system (CNS) administration (such as intraspinal, intraventricular or hypothalmic) may also be used, if desired. The compositions are typically administered to human beings, but may also be administered to animals, preferably mammals, displaying symptoms similar to autism.

### Compounds of the present invention

One or more of the components of the formulation of the present invention are tocotrienols. Tocotrienols belong to the vitamin E family and differ from the tocopherols in the chemical nature of the side chain or tail and in their properties that are clearly distinct from those of the tocopherols. Tocotrienols are fat-soluble, water-insoluble oils and modulate several mechanisms associated with the aging process and aging-related diseases.

Tocotrienols have been shown to promote healthy cholesterol levels, and exhibit neuroprotective effects, anti-oxidant activity and anti-cancer effects that are often not exhibited by tocopherols. There are three commercially used sources of tocotrienols - rice bran oil, palm oil, and annatto bean. Annatto tocotrienol extracts contain, approximately, no alpha-tocotrienol, 90% of delta-tocotrienol, 10% of gamma-tocotrienol, and no tocopherols. Rice bran extract contains, approximately, less than 2% alpha-tocotrienol, 51.6% gamma-tocotrienol, and 48% tocopherols. Palm oil extract contains, approximately, 22.1 % alpha-tocotrienol, 10% delta-tocotrienol, 45.7% gamma-tocotrienol, and 21.8% tocopherols. Tocotrienols are often used in small quantities as antioxidants to prevent decomposition and enhance the stability of other components in formulations, but in the present invention tocotrienols are used as one of the essential components of the medical food or dietary food for the prevention and amelioration of Pervasive Developmental Disorder (PDD), including of Autistic Disorder, Asperger's Disorder, Childhood Disintegrative Disorder (CDD), Rett's Disorder, and PDD-Not Otherwise Specified (PDD-NOS) in a human patient.

Some formulations of the present invention particularly comprise alpha-tocotrienol which can be produced synthetically or derived from one of the commercial sources mentioned above. A preferred process for the production of essentially pure alpha-tocotrienol has been described in co-owned US application USAN 12/606,923 titled "Process for Enrichment and Isolation of alpha-Tocotrienol and Derivatives" hereby incorporated by reference in its entirety.

Syntheses of various members of the tocotrienol family in the d,1- or (RS)-form have been published, see for example Schudel et al., Helv. Chim. Acta (1963) 46, 2517-2526; Mayer et al., Helv. Chim. Acta (1967) 50, 1376-1393; Kabbe et al., Synthesis (1978), 888-889; Kajiwara et al., Heterocycles (1980) 14, 1995-1998; Urano et al., Chem. Pharm. Bull. (1983) 31, 4341-4345; Pearce et al., J. Med Chem. (1992), 35, 3595-3606; and Pearce et al., J. Med. Chem. (1994). 37, 526-541. None of these reported processes lead to the natural form of the tocotrienols, but rather produces racemic mixtures. Syntheses of natural form d-tocotrienols have been published. See for example, Scott et al., Helv. Chim. Acta (1976) 59, 290-306; Sato *et al.,* Japanese Patent No. 63063674; Sato *et al.,* Japanese Patent No. 01233278; and Couladouros et al (US Patent No. 7,038,067).

Some other formulations of the present invention comprise tocotrienol esters selected from alpha-tocotrienol acetate, alpha-tocotrienol succinate, alpha-tocotrienol phosphate, alpha-tocotrienol aspartate, alpha-tocotrienol glutamate, alpha-tocotrienol palmitate, alpha-tocotrienol nicotinate, and polyethoxylated alpha-tocotrienol.

Some other formulations of the present invention particularly comprise tocotrienols from extracts of palm oil, rice bran oil and barley. While synthetic and natural tocopherols are readily available in the market, natural tocotrienols supply is limited, and generally comprises a mixture of tocotrienols. Crude palm oil which is rich in tocotrienols (800-1500 ppm) offers a potential source of natural tocotrienols. Carotech, Malaysia is an industrial plant that is able to extract and concentrate tocotrienols from crude palm oil, by a process patented in U.S. Pat. No. 5,157,132. Tocomin-50^{®} typically comprises about 25.32% mixed tocotrienols (7.00% alpha-tocotrienol, 14.42% gamma tocotrienol, 3.30% delta tocotrienol and 0.6% beta tocotrienol), 6.90% alpha-tocopherol and other phytonutrients such as plant squalene, phytosterols, co-enzyme Q10 and mixed carotenoids.

Some other formulations of the present invention comprise tocotrienols from extracts of annatto beans.

Additional commercially available products that may be used in the present invention are for example, Nu Triene Tocotrienol^{®} (30% content, a product of Eastman Chemical Company), various Oryza^{®} tocotrienol products of different tocotrienol concentrations from Oryza Oil & Fat Co. Ltd including Oryza tocotrienol-70 with 70% total tocopherol/tocotrienol content, and a total tocotrienol content of 40% including 14% of alpha-tocotrienol and 24% gamma-tocotrienol, and Oryza tocotrienol-90 with 90% total tocopherol/tocotrienol content and a total tocotrienol content of 60%; Golden Hope Plantations Berhad Tocotrienol oil (70% content), Davos Life Science TRF (63% content) and Palm Nutraceuticals Sdn Bhd (89% content). Delta Tocotrienol-92^{®} (92% pure by HPLC) is a commercially available product from Beijing Gingko Group that may be also used in the present invention.

Other methods for isolation or enrichment of tocotrienol from certain plant oils and plant oil by-products have been described in the literature. For some examples of such isolation and purification processes, see for instance Top et al., U.S. Pat. No. 5,190,618; Lane et al., U.S. Pat No. 6,239,171; Bellafiore et al, U.S. Pat. No. 6,395,915; May et al., U.S. Pat. No. 6,656,358; Jacobs et al., U.S. Pat. No. 6,838,104; Sumner et al. Int. Pat. Pub. WO 99/38860, Jacobs Int. Pat. Pub. WO 2002/500054, and Bhaggan et al. Int. Pat. Pub. WO 2008/142433.

### Clinical Assessment of Autism

Several biochemical markers have been associated with Pervasive Developmental Disorders. These biomarkers can be monitored during treatment with the methods and compositions of the invention, in order to assess efficacy of treatment.

*Carnitine Deficiency*: As documented by Filipek, PA et al, in J, Autism Dev. Diosrd. (2004) 34:615-23 serum carnitine levels on 100 children with autism were investigated, concurrently with serum pyruvate, lactate, ammonia, and alanine levels. Values of free and total carnitine (p < 0.001), and pyruvate (p = 0.006) were significantly reduced while ammonia and alanine levels were considerably elevated (p < 0.001) in the autistic subjects. The relative carnitine deficiency in these patients, accompanied by slight elevations in lactate and significant elevations in alanine and ammonia levels would suggest that assessing pyruvate, lactate, carnitine and ammonia levels may be useful when measured during routine evaluation of ASD children.

*Lactic acid (lactate) levels*: Some cases of autism have been associated with abnormal levels of lactic acid, as pyruvate levels increase and pyruvate is converted to lactate to maintain capacity for glycolysis (see Coleman, M. et al. Journal of Autism and Developmental Disorders (1985) 15, 1-8.) Lactate levels can be measured by taking samples of appropriate bodily fluids such as whole blood, plasma, or cerebrospinal fluid. Using magnetic resonance, lactate levels can be measured in virtually any volume of the body desired, such as the brain. Whole blood, plasma, and cerebrospinal fluid lactate levels can be measured by commercially available equipment such as the YSI 2300 STAT Plus Glucose & Lactate Analyzer (YSI Life Sciences, Ohio).

*Lipid Peroxidation*: Lipid peroxidation has been found to be elevated in autism indicating that oxidative stress is increased in this disease. Lipid peroxidation can be measured by quantifying the levels of malonyldialdehyde (MDA), an end product of fatty acid oxidation. Several assays exist for MDA in plasma, urine, and other specimens. Such assays include specific reagents for UV detection by HPLC (Steghens, J. P., et al., Free Radic Biol Med (2001) 31:242 and Pilz, J. Chromatogr B Biomed Sci appl (2000) 742:315 and capillary electrophoresis (Korizis, K. N. et al., Biomed Chromatogr 15:287 (2001)). A variety of lipid peroxidation products including MDA can be quantified using the thiobarbituric acid reaction (K Fukanaga et al., Biomed Chromatogr (1998)12:300).

Lipid peroxidation can also be quantified by measurement of urinary levels of isoprostane F(2a)-VI, a marker of lipid peroxidation; 2,3-dinor-thromboxane B(2), which reflects platelet activation; and 6-keto-prostaglandin F(1a), a marker of endothelium activation, by means of gas chromatography-mass spectrometry in subjects with autism and healthy control subjects. Methods of detecting oxidant stress-related products are likewise known in the art. For example, enzyme immunoassay kits are commercially available from Cayman Chemical for determination of isoprostane F(2a)-VI (Cayman Chemical cat. no. 516301); for 2,3-dinor-thromboxane B(2) (Cayman Chemical cat. no. 519051), and for 6-keto-prostaglandin F(1a) (Cayman Chemical cat. no. 5152111).

*Antioxidant Proteins*: Levels of major antioxidant proteins namely, transferrin (iron-binding protein) and ceruloplasmin (copper-binding protein) in the serum, are significantly reduced in autistic children as compared to their developmentally normal non-autistic siblings. A striking correlation was observed between reduced levels of these proteins and loss of previously acquired language skills in children with autism. These results indicating altered regulation of transferrin and ceruloplasmin in autistic children who lose acquired language skills can be used as diagnosis of disease during evaluation of patients.

### Pharmaceutical, nutraceutical and nutritional formulations

The compounds described herein can be formulated as pharmaceutical compositions by formulation with additives such as pharmaceutically acceptable excipients, pharmaceutically acceptable carriers, and pharmaceutically acceptable vehicles, or as nutraceutical or nutritional formulations with additives such as nutraceutically or nutritionally acceptable excipients, nutraceutically or nutritionally acceptable carriers, and nutraceutically or nutritionally acceptable vehicles.

Suitable pharmaceutically acceptable excipients, carriers and vehicles include processing agents and drug delivery modifiers and enhancers, such as, for example, calcium phosphate, magnesium stearate, talc, monosaccharides, disaccharides, starch, gelatin, cellulose, methyl cellulose, sodium carboxymethyl cellulose, dextrose, hydroxypropyl-β-cyclodextrin, polyvinylpyrrolidinone, low melting waxes, ion exchange resins, and the like, as well as combinations of any two or more thereof. Other suitable pharmaceutically acceptable excipients are described in "Remington's Pharmaceutical Sciences," Mack Pub. Co., New Jersey (1991), and "Remington: The Science and Practice of Pharmacy," Lippincott Williams & Wilkins, Philadelphia, 20th edition (2003) and 21st edition (2005), incorporated herein by reference.

A pharmaceutical composition can comprise a unit dose formulation, where the unit dose is a dose sufficient to have a therapeutic or suppressive effect. The unit dose may be sufficient as a single dose to have a therapeutic or suppressive effect. Alternatively, the unit dose may be a dose administered periodically in a course of treatment or suppression of a disorder. A unit dose can contain a therapeutically effective amount of a composition disclosed herein. Alternatively, a unit dose can contain a physiologically effective amount of a composition disclosed herein.

Pharmaceutical compositions containing the compounds of the invention may be in any form suitable for the intended method of administration, including, for example, a solution, a suspension, or an emulsion. Liquid carriers are typically used in preparing solutions, suspensions, and emulsions. Liquid carriers contemplated for use in the practice of the present invention include, for example, water, saline, pharmaceutically acceptable organic solvent(s), pharmaceutically acceptable oils or fats, and the like, as well as mixtures of two or more thereof. The liquid carrier may contain other suitable pharmaceutically acceptable additives such as solubilizers, emulsifiers, nutrients, buffers, preservatives, suspending agents, thickening agents, viscosity regulators, stabilizers, and the like. Suitable organic solvents include, for example, monohydric alcohols, such as ethanol, and polyhydric alcohols, such as glycols. Suitable oils include, for example, soybean oil, coconut oil, olive oil, safflower oil, cottonseed oil, and the like. For parenteral administration, the carrier can also be an oily ester such as ethyl oleate, isopropyl myristate, and the like. Compositions of the present invention may also be in the form of microparticles, microcapsules, liposomal encapsulates, and the like, as well as combinations of any two or more thereof.

The compositions, as described above, can be prepared as nutritional formulations such as foods, including medical or functional foods and dietary supplements. A "medical or functional food" is defined as being consumed as part of a usual diet which has been demonstrated to have physiological benefits and/or to reduce the risk of chronic disease beyond basic nutritional functions. A "dietary supplement" is defined as a product that is intended to supplement the human diet and is typically provided in the form of a pill, capsule, tablet, or like formulation. By way of example, but not limitation, a dietary supplement may include one or more of the following ingredients: vitamins, minerals, herbs, botanicals, amino acids, dietary substances intended to supplement the diet by increasing total dietary intake, and concentrates, metabolites, constituents, extracts or combinations of any of the foregoing. Dietary supplements may also be incorporated into food stuffs, such as functional foods designed to promote health or to prevent disease or disorders. If administered as a medicinal preparation, the composition can be administered, either as a prophylaxis or treatment, to a patient in any of a number of methods. The subject compositions may be administered alone or in combination with other pharmaceutical agents and can be combined with a physiologically acceptable carrier thereof. The effective amount and method of administration of the particular formulation can vary based on the individual subject, the stage of disease, and other factors evident to one skilled in the art. During the course of the treatment, the concentration of the subject compositions may be monitored (for example, blood plasma levels may be monitored) to insure that the desired level is maintained.

The term "nutraceutical" has been used to refer to any substance that is a food or a part of a food and provides medical or health benefits, including the prevention and treatment of disease. Hence, compositions falling under the label "nutraceutical" may range from isolated nutrients, dietary supplements and specific diets to genetically engineered designer foods, herbal products, and processed foods such as cereals, soups and beverages. In a more technical sense, the term has been used to refer to a product isolated or purified from foods, and generally sold in medicinal forms not usually associated with food and demonstrated to have a physiological benefit or provide protection against chronic disease. Suitable nutraceutically acceptable excipients may include liquid solutions such as a solution comprising a vegetable- and/or animal- and/or fish-derived oil.

The compositions of the present invention can be dissolved in a vegetable oil that is appropriate as a food additive such as soybean oil, sunflower oil, olive oil or other similar oils derived usually from seeds. The dispersion or emulsion can be dispersed or spray dried and agglomerated.

The compositions of the present invention can be administered in the form of an emulsified liquid solution that can be delivered as concentrates or in unit dose packaging for immediate consumption. Some emulsifiers described in US Pat. 7,118,688 may be sorbitol egg yolk phospholipids, and sorbitol and soybean phospholipids, optionally comprising flavors such as chocolate. Other examples of emulsifiers include, but are not limited to lecithin, preferably natural soy lecithin, polyoxyethylene stearate, polyoxyethylene sorbitan mono-oleate, polyoxyethylene sorbitan monopalmitate, polyoxyethylene sorbitan monostearate, polyoxyethylene sorbitan monolaurate, ammonium phosphatides, citric acid esters of mono- or di-glycerides of fatty acids, and tartaric acid esters of mono- or di-glycerides of fatty acids. The emulsifier should be one that is effective at achieving an oil-in-water emulsion.

In one embodiment, the emulsion is formulated as a powder, wherein said emulsion is mixed with a powder substance and the formed suspension is spray dried or freeze dried. Processes for such formulations are known in the art, for example as described by Yokoi et al., US Pat. No. 6,562,372. Dextrins such as cyclodextrin or maltodextrin may be added for the pulverization of the emulsion.

The powder composition may contain a desired amount of carbohydrates. Water-based food compositions typically include between about 5.0% to about 35% by weight of carbohydrates. An exemplary carbohydrate is Maltodextrin DE 10 because it provides a mild sweetener combined with a good source of carbohydrates. Maltodextrins are derived from corn starches. They are classified by dextrose equivalent or DE, which is a measure of the reducing sugars present calculated as dextrose and expressed as a percentage of the total dry substance. Maltodextrins can go up to 20 DE. At and above 20 DE, the product is classified as Corn Syrup Solids. The lower the DE, the less sweet and the more like starch the maltodextrin is. As the DE decreases from 20 to 1, the maltodextrins also decrease in solubility. Above a DE of 20, the product is corn syrup solids, is completely soluble, and therefore imparts significant sweetness.

Alternatively the formulation of the present invention can be administered in the form of a beverage product. The liquid beverage of the invention may be prepared by the individual at ambient temperature by dissolving for immediate consumption the powder as described above, in a consumable liquid, such as water, milk, carbonated drink, and fruit juice. Liquid compositions for oral administration prepared in water or other aqueous consumable vehicles can include solutions, emulsions, syrups and elixirs containing together with the active compounds, wetting agents, sweeteners, coloring agents and flavoring agents. Alternatively the formulation of the present invention can be administered in the form of a beverage product ready to be consumed.

The formulation of the present invention can be administered in the form of a high protein food bar that may be used to supplement protein intake particularly in individuals in need of such supplementation. The formulation may be in the form of ready-to-eat packages. The formulation administered in the form of a high protein food bar may contain carbohydrates and dietary fibers.

The term "protein source" includes but is not limited to, plant proteins, animal proteins, proteins from single cell organisms and free amino acids. Sources of protein for high protein food bars include, but are not limited to, proteins derived from milk, whey, beef, egg, legumes, peanut wheat, soy and combinations thereof, such as whey protein, soy protein, casein, hydrolyzed beef protein, hydrogenated peanut oil, and combinations thereof.

The term "carbohydrate" includes simple (mono and disaccharides) and complex (polysaccharides) carbohydrates. The simple carbohydrates may be any of the digestible carbohydrates such as dextrose, fructose, high fructose corn syrup, sucrose, maltose, high maltose corn syrup, maltodextrin, lactose, glucose, oligosaccharides, high saccharides, corn starch, cellulose, or mixtures thereof, depending on usage. Complex carbohydrates are provided by but not limited to sources as cereal grains such as wheat, oat, corn, barley, rice, rye, sorghum; legumes both mature and dry, such as soybeans; and nuts such as peanuts, and the like. Cereal grains may also act as sources of fiber, may be rolled, toasted, extruded and otherwise treated to add to the chew texture.

The carbohydrates can be in the form of grains, flakes, flours, and meals. Simple carbohydrates including fructose should constitute from about 30-60% of the carbohydrates. Complex carbohydrates should constitute from about 40-70% of the carbohydrates. The blend of carbohydrates including fibers is selected to add to sustained energy.

Dietary fiber can be divided into two broad categories: insoluble dietary fiber and water soluble dietary fiber. Best suited are cereal bran and mixtures thereof due to their relatively high insoluble dietary fiber content. Those cereal brans useful in this invention are selected from the group consisting of rice, wheat, corn, barley, rye, oats, pea and mixtures thereof. Wheat, oat and corn bran are the most preferred. The components of the insoluble dietary fiber derived from these brans are known to be cellulose, hemicellulose and lignin.

The soluble dietary fibers may be film-forming hydrocolloid materials such as alginates, gums, pectin, mucilages and similar plant exudates. Examples of useful soluble fibers are arabic, tragacanth, karaya gum, ghatti gum, seaweed extracts including agar, alginates, carrageenans, and furcellaran; pectin; and mucilages such as psyllium. Dietary fiber should constitute about 2-15% of the carbohydrates. The ratio of insoluble to soluble fiber can range from 50:50 to about 99:1 with ratios in the range of about 80:20 to about 99:1 preferred. The blend of higher insoluble to soluble also adds to the improved taste characteristics of the food bar.

Another delivery form of the formulations of the present invention is packaged as a mixture, preferably microencapsulated, in small impermeable, disposable packages such as packets (e.g., 11/2" x 2" in size) or small tubes (e.g., 1/4" diameter x 2" in length) which may be foil, plastic, or other disposable material under inert conditions. In some of these configurations, the contents of the packages containing the formulations are administered to the patient directly from the package or mixed with food or a cold consumable liquid. In another delivery form the solid composition of the present invention is packaged in nitrogen sealed cans.

Time-release or controlled release delivery systems may be used, such as a diffusion controlled matrix system or an erodible system, as described for example in: Lee, "Diffusion-Controlled Matrix Systems", pp. 155-198 and Ron and Langer, "Erodible Systems", pp. 199-224, in "Treatise on Controlled Drug Delivery", A. Kydonieus Ed., Marcel Dekker, Inc., New York 1992. The matrix may be, for example, a biodegradable material that can degrade spontaneously *in situ* and *in vivo* for, example, by hydrolysis or enzymatic cleavage, e.g., by proteases. The delivery system may be, for example, a naturally occurring or synthetic polymer or copolymer, for example in the form of a hydrogel. Exemplary polymers with cleavable linkages include polyesters, polyorthoesters, polyanhydrides, polysaccharides, poly(phosphoesters), polyamides, polyurethanes, poly(imidocarbonates) and poly(phosphazenes).

The compounds of the invention may be administered enterally, orally, parenterally, sublingually, by inhalation (e.g. as mists or sprays), rectally, or topically in dosage unit formulations containing conventional nontoxic pharmaceutically acceptable carriers, adjuvants, and vehicles as desired. For example, suitable modes of administration include oral, subcutaneous, transdermal, transmucosal, iontophoretic, intravenous, intraarterial, intramuscular, intraperitoneal, intranasal (e.g. via nasal mucosa), subdural, rectal, gastrointestinal, and the like, and directly to a specific or affected organ or tissue. For delivery to the central nervous system, spinal and epidural administration, or administration to cerebral ventricles, can be used. Topical administration may also involve the use of transdermal administration such as transdermal patches or iontophoresis devices. The term parenteral as used herein includes subcutaneous injections, intravenous, intramuscular, intrasternal injection, or infusion techniques. The compounds are mixed with pharmaceutically acceptable carriers, adjuvants, and vehicles appropriate for the desired route of administration. Oral administration is a preferred route of administration, and formulations suitable for oral administration are preferred formulations. The compounds described for use herein can be administered in solid form, in liquid form, in aerosol form, or in the form of tablets, pills, powder mixtures, capsules, granules, injectables, creams, solutions, suppositories, enemas, colonic irrigations, emulsions, dispersions, food premixes, and in other suitable forms. The compounds can also be administered in liposome formulations. The compounds can also be administered as prodrugs, where the prodrug undergoes transformation in the treated subject to a form which is therapeutically effective. Additional methods of administration are known in the art.

Injectable preparations, for example, sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, for example, as a solution in propylene glycol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables.

Solid dosage forms for oral administration may include capsules, tablets, pills, powders, and granules. In such solid dosage forms, the active compound may be admixed with at least one inert diluent such as sucrose, lactose, or starch. Such dosage forms may also comprise additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., Methods in Cell Biology, Volume XIV, Academic Press, New York, N.W., p. 33 et seq (1976).

In a preferred embodiment, the compositions are provided to the patient as either a medical or dietary food or a food supplement. For example, when provided as a food the compositions of the present invention are combined with material primarily made up of protein, carbohydrate and/or fat that is used in the body, preferably a human body, to sustain growth, repair, vital processes, and to furnish energy. When provided as a food supplement, the compositions comprise selected substances such that they can be eaten at or about the same time as a food. The food supplements are generally eaten within about one hour before or after the food is eaten, typically within about one-half hour before or after the food is eaten, preferably within about 15 minutes of when the food is eaten, and further preferably within one to five minutes of the time the food is eaten. The food supplement can also be eaten at the same time as, or even with the food.

The invention also provides articles of manufacture and kits containing materials useful for treating or suppressing PDD including Autism or for reducing the symptoms of one or more symptoms of PDD. The invention also provides kits comprising of a tocotrienol, a tocotrienol ester, a tocotrienol ether, a tocotrienol enriched extract or composition, or mixtures thereof. In some embodiments, the kit of the invention comprises the container described above.

In other aspects, the kits may be used for any of the methods described herein, including, for example, to treat an individual with a Pervasive Developmental Disorder, including an autistic spectrum disorder, or to suppress a Pervasive Developmental Disorder, such as an autistic spectrum disorder in an individual.

The amount of active ingredient that may be combined with the carrier materials to produce a single dosage form will vary depending upon the host to which the active ingredient is administered and the particular mode of administration. It will be understood, however, that the specific dose level for any particular patient will depend upon a variety of factors including the activity of the specific compound employed, the age, body weight, body area, body mass index (BMI), general health, sex, diet, time of administration, route of administration, rate of excretion, drug combination, and the type, progression, and severity of the particular disease undergoing therapy. The pharmaceutical unit dosage chosen is usually fabricated and administered to provide a defined final concentration of drug in the blood, tissues, organs, or other targeted region of the body. The therapeutically effective amount or effective amount for a given situation can be readily determined by routine experimentation and is within the skill and judgment of the ordinary clinician. The composition can be administered once a day, twice a day or three times a day. The composition can be administered at the recommended maximum clinical dosage or at lower doses. Dosage levels of the active compounds in the formulation of the invention may be varied so as to obtain a desired therapeutic response depending on the severity of the disease and the response of the patient.

Examples of dosages which can be used are an effective amount within the dosage range of about 0.1 mg/kg to about 500 mg/kg body weight, 0.1 mg/kg to about 300 mg/kg body weight, or within about 1.0 mg/kg to about 100 mg/kg body weight, or within about 1.0 mg/kg to about 50 mg/kg body weight, or within about 1.0 mg/kg to about 30 mg/kg body weight, or within about 1.0 mg/kg to about 10 mg/kg body weight, or within about 10 mg/kg to about 100 mg/kg body weight, or within about 50 mg/kg to about 150 mg/kg body weight, or within about 100 mg/kg to about 200 mg/kg body weight, or within about 150 mg/kg to about 250 mg/kg body weight, or within about 200 mg/kg to about 300 mg/kg body weight, or within about 250 mg/kg to about 300 mg/kg body weight. Compounds of the present invention may be administered in a single daily dose, or the total daily dosage may be administered in divided dosage of two, three or four times daily.

While the compounds of the invention can be administered as the sole active pharmaceutical agent, they can also be used in combination with one or more other agents used in the treatment or suppression of disorders. If compounds are administered together, they need not be administered by the same route, or in the same formulation. However, they can be combined into one formulation as desired.

In some embodiments, representative agents are useful in combination with the compounds and methods of the invention for the treatment or suppression of PDD or ASD symptoms include, but are not limited to, antioxidant compounds and/or drugs, such as but not limited to carnitine, quercetine, mangosteen, acai, uridine, N-acetyl cysteine (NAC), and glutathione.

In other embodiments, representative agents useful in combination with the compounds and methods of the invention for the treatment or suppression of PDD or ASD symptoms include one or more compounds selected from alpha-lipoic acid, L-carnitine or acetyl L-carnitine, Vitamin C, at least one of the B vitamins selected from vitamin B 1 (Thiamine), vitamin B2 (Riboflavin), vitamin B3 (Niacin), vitamin B4 (Niacinamide), vitamin B5 (Pantothenic acid), vitamin B6 (Pyridoxine), vitamin B8 (Inositol), vitamin B9 (Folic acid), vitamin B10 (PABA) and vitamin B12 (Cobalamins), N-acetyl cysteine (NAC), leucine, creatine, taurine and caffeine.

Other agents useful in combination with the compounds and methods of the invention are compounds and/or drugs that have an effect on the neurotransmitters, particularly serotonin and dopamine, that include antidepressants, anti-anxiety drugs, antispasmodics, neuroleptics and atypical neuroleptics, and stimulants. Antidepressants include but are not limited to Selective Serotonin Reuptake Inhibitors (SSRIs) (fluoxetine (Prozac); fluvoxamine (Luvox); paroxetine (Paxil); sertraline (Zoloft); citalopram (Celexa)); Tricyclic antidepressants (amitriptyline (Elavil); amitriptyline/chlordiazepoxide (Limbitrol); amoxapine (Asendin); clomipramine (Anafranil); desipramine (Norpramin); doxepin (Sinequan); imipramine (Tofranil); nortriptyline (Avenytl, Pamelor); protriptyline (Vivactil); trimipramine (Surmontil)); MAO Inhibitors (moclobemide (Aurorex); phenelzine (Nardil); tranylcypromine sulfate (Parnate)); Buproprion; Lithium; Mirtazapine; Nefazodone (Serzone); Reboxetine (Edronax); Venlafaxine (Effexor, Effexor XR); and "natural" antidepressants such as St. John's Wart. Anti-anxiety drugs include but are not limited to alprazolam (Xanax); chlordiazepoxide (Librium); clonazepam (Klonopin); clorazepate (Tranxene); diazepam (Valium); lorazepam (Ativan); oxazepam (Serax); and prazepam (Centrax). Antispasmodic medications include but are not limited to carmazepine (Tegretol); clonazepam (Klonopin); ethosuximide (Zarontin); ethotoin (Peganone); fosphenytoin (Cerebyx); gabapentin (Neurontin); lamotrigine (Lamictal); mephenytoin (Mesantoin); phenobarbital (Luminol, Solfoton); phenytoin (Dilantin); primidone (Mysoline); toiramate (Topamax); valproic acid (Depakene); divalproex sodium (Depakote, Depakote Sprinkles); and Gabapentin (Neurontin). Stimulants include but are not limited to dextroamphetamine sulfate (Das, Dexampex, Dexedrine, Dexedrine Spansules, Dextrostat, Ferndex, Oxydess); dextroamphetamine/amphetamine (Adderall); methamphetamine (MTH); methylphenidate hydrochloride (Ritalin); and pemoline (Cyclert); and phenylpropanolamine (PPA). Atypical neuroleptics include but are not limited to clozapine (Clozaril); olanzapine (Zyprexa); risperidone (Risperdal); quetiapine (Seroquel); and ziprasidone (Zeldox).

The compounds disclosed herein for use in methods of the invention can be administered in combination with one or more minerals, such as, but not limited to, iron, calcium, potassium, zinc, manganese, phosphorous, chromium, magnesium, manganese, molybdenum, tin, nickel, sulfur, selenium, copper, cobalt, chloride, fluoride or iodide. The minerals may be administered in large or trace amounts. The compounds can be administered together with the minerals in one formulation or in different formulations.

When additional active agents are used in combination with the compounds and methods of the present invention, the additional active agents may generally be employed in therapeutic amounts as indicated in the Physicians' Desk Reference (PDR) 53rd Edition (1999), or such therapeutically useful amounts as would be known to one of ordinary skill in the art.

The compounds of the invention and the other therapeutically active agents disclosed herein for use in methods of the invention can be administered at the recommended maximum clinical dosage or at lower doses. Dosage levels of the active compounds in the compositions of the invention may be varied so as to obtain a desired therapeutic response depending on the route of administration, severity of the disease and the response of the patient. When administered in combination with other therapeutic agents, the therapeutic agents can be formulated as separate compositions that are given at the same time or different times, or the therapeutic agents can be given as a single composition.

The invention will be further understood by the following non-limiting example.

### Example

### Screening Compounds of the Invention in Human Dermal Fibroblasts from Autistic Patients

A screen was performed to identify compounds effective for the amelioration of ASD. Test samples, and solvent controls were tested for their ability to rescue ASD fibroblasts stressed by addition of L-buthionine-(S,R)-sulfoximine (BSO).

MEM (a medium enriched in amino acids and vitamins, catalog no. Gibco 11965) and Fetal Calf Serum were obtained from Invitrogen. Basic fibroblast growth factor and epidermal growth factor were purchased from PeproTech. Penicillin-streptomycin-glutamine mix, L-buthionine (S,R)-sulfoximine, and insulin from bovine pancreas were purchased from Sigma. Calcein AM was purchased from Molecular Probes. Cell culture medium (ATP) was made by combining 75 ml Fetal Calf Serum, 100 U/ml penicillin, 100 µg/ml streptomycin, 2 mM glutamine, 10 ng/ml EGF, and 10 ng/ml bFGF; MEM EBS was added to make the volume up to 500 ml. A 10 mM BSO solution was prepared by dissolving 444 mg BSO in 200 ml of medium with subsequent filter-sterilization. During the course of the experiments, this solution was stored at +4°C. The cells were obtained from Dr. J.M. Shoffner , Medical Neurogenetics, Atlanta, GA and were grown in 10 cm tissue culture plates. Every week, they were split at a 1:3 ratio.

The samples were supplied in 1.5 ml glass vials. The compounds were diluted with DMSO, ethanol or PBS to result in a 5 mM stock solution. Once dissolved, they were stored at -20°C.

The samples were screened according to the following protocol: A culture with ASD fibroblasts was started from a 1 ml vial with approximately 500,000 cells stored in liquid nitrogen. Cells were propagated in 10 cm cell culture dishes by splitting every week in a ratio of 1:3 until nine plates were available. Once confluent, fibroblasts were harvested. For 54 micro titer plates (96 well-MTP) a total of 14.3 million cells (passage eight) were re-suspended in 480 ml medium, corresponding to 100 µl medium with 3,000 cells/well. The remaining cells were distributed in 10 cm cell culture plates (500,000 cells/plate) for propagation. The plates were incubated overnight at 37°C in an atmosphere with 95% humidity and 5% CO₂ to allow attachment of the cells to the culture plate.

MTP medium (243 µl) was added to a well of the microtiter plate. The test compounds were unfrozen, and 7.5 µl of a 5 mM stock solution was dissolved in the well containing 243 µl medium, resulting in a 150 µM master solution. Serial dilutions from the master solution were made. The period between the single dilution steps was kept as short as possible (generally less than 1 second).

Plates were kept overnight in the cell culture incubator. The next day, 10 µl of a 10 mM BSO solution were added to the wells, resulting in a 1 mM final BSO concentration. Forty-eight hours later, three plates were examined under a phase-contrast microscope to verify that the cells in the 0% control (wells E1-H1) were clearly dead. The medium from all plates was discarded, and the remaining liquid was removed by gently tapping the plate inversed onto a paper towel.

100 µl of PBS containing 1.2 µM Calcein AM were then added to each well. The plates were incubated for 50-70 minutes at room temperature. After that time the PBS was discarded, the plate gently tapped on a paper towel and fluorescence (excitation/emission wavelengths of 485 nm and 525 nm, respectively) was read on a Gemini fluorescence reader. Data was imported into Microsoft Excel (EXCEL is a registered trademark of Microsoft Corporation for a spreadsheet program) and used to calculate the EC₅₀ concentration for each compound.

The compounds were tested three times, i.e., the experiment was performed three times, the passage number of the cells increasing by one with every repetition.

The solvents (DMSO, ethanol, PBS) neither had a detrimental effect on the viability of non-BSO treated cells nor did they have a beneficial influence on BSO-treated fibroblasts even at the highest concentration tested (1%). None of the compounds showed auto-fluorescence. The viability of non-BSO treated fibroblasts was set as 100%, and the viability of the BSO- and compound-treated cells was calculated as relative to this value. Alpha-tocotrienol exhibited protection against ASD with an EC₅₀ of less than about 50 nM.

The disclosures of all publications, patents, patent applications and published patent applications referred to herein by an identifying citation are hereby incorporated herein by reference in their entirety.

### EMBODIMENTS OF THE INVENTION

The following embodiments are disclosed:
1. A method of reducing the symptoms associated with, or of treating or suppressing Pervasive Developmental Disorders (PDDs) in a patient in need of such treatment, comprising administering a therapeutically or physiologically effective amount of a composition comprising tocotrienols, tocotrienol esters, tocotrienol ethers, tocotrienol enriched extracts, or mixtures thereof.
2. The method according to embodiment 1, wherein the patient in need of such treatment exhibits symptoms associated with Autistic Disorder, Asperger's Disorder, Childhood Disintegrative Disorder (CDD), Rett's Disorder, and PDD-Not Otherwise Specified (PDD-NOS).
3. The method according to embodiment 1, wherein the composition comprises a therapeutically effective amount of one or more agents selected from the group consisting of alpha-tocotrienol, beta-tocotrienol, gamma-tocotrienol, delta-tocotrienol and mixtures thereof.
4. The method according to embodiment 3, wherein the composition comprises a therapeutically effective amount of alpha-tocotrienol.
5. The method according to embodiment 1, wherein the composition comprises a therapeutically effective amount of one or more agents selected from tocotrienol acetate, tocotrienol succinate, tocotrienol phosphate, tocotrienol aspartate, tocotrienol glutamate, tocotrienol palmitate, tocotrienol nicotinate, and polyethoxylated tocotrienol.
6. The method according to embodiment 5, wherein the composition comprises a therapeutically effective amount of one or more agents selected from alpha-tocotrienol acetate, alpha-tocotrienol succinate, alpha-tocotrienol phosphate, alpha-tocotrienol aspartate, alpha-tocotrienol glutamate, alpha-tocotrienol palmitate, alpha-tocotrienol nicotinate, and polyethoxylated alpha-tocotrienol.
7. The method according to embodiment 1, where the composition comprises a therapeutically effective amount of a tocotrienol enriched extract.
8. A medical or dietary food for reducing the symptoms associated with Pervasive Developmental Disorders (PDDs) in a patient in need of such treatment, said medical or dietary food comprising a therapeutically or physiologically effective amount of a composition comprising tocotrienols, tocotrienol esters, tocotrienol ethers, tocotrienol enriched extracts, or mixtures thereof.
9. The medical or dietary food according to embodiment 8, wherein the patient in need of such treatment exhibits symptoms associated with Autistic Disorder, Asperger's Disorder, Childhood Disintegrative Disorder (CDD), Rett's Disorder, and PDD-Not Otherwise Specified (PDD-NOS).
10. The method or dietary food according to embodiment 8, wherein the medical or dietary food comprises a therapeutically effective amount of one or more agents selected from the group consisting of alpha-tocotrienol, beta-tocotrienol, gamma-tocotrienol, delta-tocotrienol and mixtures thereof.
11. The method or dietary food according to embodiment 10, wherein the medical or dietary food comprises a therapeutically effective amount of alpha-tocotrienol.
12. The method or dietary food according to embodiment 8, wherein the medical or dietary food comprises a therapeutically effective amount of one or more agents selected from tocotrienol acetate, tocotrienol succinate, tocotrienol phosphate, tocotrienol aspartate, tocotrienol glutamate, tocotrienol palmitate, tocotrienol nicotinate, and polyethoxylated tocotrienol.
13. The method or dietary food according to embodiment 12, wherein the medical or dietary food comprises a therapeutically effective amount of one or more agents selected from alpha-tocotrienol acetate, alpha-tocotrienol succinate, alpha-tocotrienol phosphate, alpha-tocotrienol aspartate, alpha-tocotrienol glutamate, alpha-tocotrienol palmitate, alpha-tocotrienol nicotinate, and polyethoxylated alpha-tocotrienol.
14. The medical or dietary food according to embodiment 8, wherein the medical or dietary food comprises a therapeutically effective amount of a tocotrienol enriched extract.
15. Use of a therapeutically or physiologically effective amount of a composition comprising tocotrienols, tocotrienol esters, tocotrienol ethers, tocotrienol enriched extracts, or mixtures thereof for reducing the symptoms associated with, or for treating or suppressing Pervasive Developmental Disorders (PDDs) in a patient in need of such treatment.
16. The use according to embodiment 15, wherein the patient in need of such treatment exhibits symptoms associated with Autistic Disorder, Asperger's Disorder, Childhood Disintegrative Disorder (CDD), Rett's Disorder, and PDD-Not Otherwise Specified (PDD-NOS).
17. The use according to embodiment 15, wherein the composition comprises a therapeutically effective amount of one or more agents selected from the group consisting of alpha-tocotrienol, beta-tocotrienol, gamma-tocotrienol, delta-tocotrienol and mixtures thereof
18. The use according to embodiment 17, wherein the composition comprises a therapeutically effective amount of alpha-tocotrienol.
19. The use according to embodiment 15, wherein the composition comprises a therapeutically effective amount of one or more agents selected from alpha-tocotrienol acetate, alpha-tocotrienol succinate, alpha-tocotrienol phosphate, alpha-tocotrienol aspartate, alpha-tocotrienol glutamate, alpha-tocotrienol palmitate, alpha-tocotrienol nicotinate, and polyethoxylated alpha-tocotrienol.
20. The use according to embodiment 15, wherein the composition comprises a therapeutically effective amount of a tocotrienol enriched extract.

## Claims

1. A composition comprising a tocotrienol, a tocotrienol ester, a tocotrienol ether, a tocotrienol enriched extract, or a mixture thereof, for use in reducing the symptoms associated with, or in treating or suppressing, a Pervasive Developmental Disorder (PDD) in a patient in need of such treatment.

2. A composition according to claim 1, for use as defined in said claim, wherein the patient in need of such treatment exhibits symptoms associated with Autistic Disorder, Asperger's Disorder, Childhood Disintegrative Disorder (CDD), Rett's Disorder, or PDD-Not Otherwise Specified (PDD-NOS).

3. A composition according to claim 1, for use as defined in said claim, wherein the composition comprises a therapeutically effective amount of one or more agents selected from alpha-tocotrienol, beta-tocotrienol, gamma-tocotrienol, delta-tocotrienol and mixtures thereof.

4. A composition according to claim 3, for use as defined in said claim, wherein the composition comprises a therapeutically effective amount of alpha-tocotrienol.

5. A composition according to claim 1, for use as defined in said claim, wherein the composition comprises a therapeutically effective amount of one or more agents selected from tocotrienol acetate, tocotrienol succinate, tocotrienol phosphate, tocotrienol aspartate, tocotrienol glutamate, tocotrienol palmitate, tocotrienol nicotinate, and polyethoxylated tocotrienol.

6. A composition according to claim 5, for use as defined in said claim, wherein the composition comprises a therapeutically effective amount of one or more agents selected from alpha-tocotrienol acetate, alpha-tocotrienol succinate, alpha-tocotrienol phosphate, alpha-tocotrienol aspartate, alpha-tocotrienol glutamate, alpha-tocotrienol palmitate, alpha-tocotrienol nicotinate, and polyethoxylated alpha-tocotrienol.

7. A composition according to claim 1, for use as defined in said claim, wherein the composition comprises a therapeutically effective amount of a tocotrienol enriched extract.

8. A medical or dietary food for use in reducing the symptoms associated with a Pervasive Developmental Disorder (PDD) in a patient in need of such treatment, said medical or dietary food comprising a therapeutically or physiologically effective amount of a composition comprising a tocotrienol, a tocotrienol ester, a tocotrienol ether, a tocotrienol enriched extract, or a mixture thereof.

9. A medical or dietary food according to claim 8, for use as defined in said claim, wherein the patient in need of such treatment exhibits symptoms associated with Autistic Disorder, Asperger's Disorder, Childhood Disintegrative Disorder (CDD), Rett's Disorder, or PDD-Not Otherwise Specified (PDD-NOS).

10. A medical or dietary food according to claim 8, for use as defined in said claim, wherein the medical or dietary food comprises a therapeutically effective amount of one or more agents selected from alpha-tocotrienol, beta-tocotrienol, gamma-tocotrienol, delta-tocotrienol and mixtures thereof.

11. A medical or dietary food according to claim 10, for use as defined in said claim, wherein the medical or dietary food comprises a therapeutically effective amount of alpha-tocotrienol.

12. A medical or dietary food according to claim 8, for use as defined in said claim, wherein the medical or dietary food comprises a therapeutically effective amount of one or more agents selected from tocotrienol acetate, tocotrienol succinate, tocotrienol phosphate, tocotrienol aspartate, tocotrienol glutamate, tocotrienol palmitate, tocotrienol nicotinate, and polyethoxylated tocotrienol.

13. A medical or dietary food according to claim 12, for use as defined in said claim, wherein the medical or dietary food comprises a therapeutically effective amount of one or more agents selected from alpha-tocotrienol acetate, alpha-tocotrienol succinate, alpha-tocotrienol phosphate, alpha-tocotrienol aspartate, alpha-tocotrienol glutamate, alpha-tocotrienol palmitate, alpha-tocotrienol nicotinate, and polyethoxylated alpha-tocotrienol.

14. A medical or dietary food according to claim 8, for use as defined in said claim, wherein the medical or dietary food comprises a therapeutically effective amount of a tocotrienol enriched extract.

15. A medical or dietary food suitable for reducing the symptoms associated with a Pervasive Developmental Disorder (PDD) in a patient in need of such treatment, said medical or dietary food comprising a therapeutically or physiologically effective amount of a composition comprising a tocotrienol, a tocotrienol ester, a tocotrienol ether, a tocotrienol enriched extract, or a mixture thereof.
